Europäisches Patentamt

European Patent Office

Office européen des brevets

(19)

(11) Publication number: **0 368 808**

**A2**

(12)

# EUROPEAN PATENT APPLICATION

(21) Application number: 89810831.1

(51) Int. Cl.5: **C12N 11/10, C12P 19/34**

(22) Date of filing: 03.11.89

(30) Priority: 07.11.88 JP 280590/88

(43) Date of publication of application:
16.05.90 Bulletin 90/20

(84) Designated Contracting States:
AT BE CH DE ES FR GB GR IT LI LU NL SE

(71) Applicant: NIPPON SHINYAKU COMPANY,
LIMITED
14, Kisshoin Nishinosho Monguchicho
Minami-ku Kyoto-shi Kyoto 601(JP)

(72) Inventor: Kise, Masahiro
Tominokoji Higashiiru Aneyakoji
Nakakyo-ku Kyoto(IT)
Inventor: Ohkubo, Takashi
A-9 Mikami Omifuji Danchi Yasucho
Shige-ken(JP)
Inventor: Konno, Kiyotaka
2-9-9-304, Nishisakaidanicho
Nishikyo-ku Kyoto(JP)
Inventor: Tomi, Hironori
703, Opasu Shiojo Ayanokoji Higashinotoin
Higashiiiru Shinmeicho Shimokyo-ku
Kyoto(JP)

(74) Representative: Kügele, Bernhard et al
c/o NOVAPAT-CABINET CHEREAU 9, Rue du
Valais
CH-1202 Genève(CH)

(54) Immobilized enzyme and a method for application thereof.

(57) Porous chitosan beads are used as carriers for immobilization of polynucleotide phosphorylase (PNPase). Immobilization of PNPase to the porous chitosan bead carrier can be effected by contacting the enzyme with the carrier in water or in a buffer solution such as phosphate or borate buffer solution, preferably at room temperature. The thus obtained immobilized enzyme is contacted with a solution of nucleoside, e.g. cytidine-5'-diphosphate or inosine-5'-diphosphate, to produce the corresponding polynucleotide, e.g. poly C or poly I. The yield of polynucleotide is thus improved and the costs reduced. Also, the reaction can be conducted by a continuous process and the chain length of the polynucleotide can be controlled.

# IMMOBILIZED ENZYME AND A METHOD FOR APPLICATION THEREOF

Detailed Description of the Invention

(Field of Industrial Application)

The present invention relates to an immobilized enzyme useful for industrially producing medical drugs and a process for producing polynucleotide using the same. More particularly, the present invention relates to an immobilized enzyme comprising porous chitosan beads having immobilized thereto polynucleotide phosphorylase (hereinafter simply referred to as PNPase) and a process for producing polynucleotide using the same.

That is, the present invention relates to:

(1) An immobilized enzyme comprising porous chitosan beads having immobilized thereto polynucleotide phosphorylase.

(2) A process for producing polynucleotide characterized by using an immobilized enzyme as claimed in claim 1.

(3) An immobilized enzyme comprising porous chitosan beads, which undergo a crosslinking treatment with a crosslinking agent, having immobilized thereto polynucleotide phosphorylase.

(4) A process for producing polynucleotide characterized by using an immobilized enzyme as claimed in claim 4.

(Prior Art)

It is well known that PNPase acts on nucloeoside-5$'$-diphosphate in the presence of $Mg^{2+}$ or $Mn^{2+}$ to isolate inorganic phosphate giving polynucleotide. When, for example, cytidine-5$'$-diphosphate (hereafter CDP) is used as nucloeoside-5$'$-diphosphate in this reaction as a substrate, polynucleotide C (hereafter poly C) is synthesized; when inosine -5$'$-diphosphate (hereafter IDP) is used as a substrate, polynucleotide I (hereafter poly I) is synthesized. These poly C and poly I are extremely useful as raw materials for poly I.C which is known to be an interferon inducer.

In producing polynucleotide, there is performed a so-called batch reaction which comprises adding PNPase to a solution of nucleoside to perform a synthetic reaction, denaturating and inactivating PNPase with phenol, chloroform, butanol, a surface active agent, etc. after completion of the reaction and then purifying polynucleotide.

On the other hand, there are known immobilized enzymes comprising (1) DEAE cellulose (J. Ferment. Technol., 64, 517-522, 1986), (2) agarose gel (Febs Letters 30, 246-248), (3) CPG (Controlled pPore Glass) (same as in (2)) having immobilized thereto PNPase.

(Problems to be solved by the Invention)

The immobilized enzymes described above cause an increase in viscosity and suffer loss due to pressure so that it is impossible to conduct continuous reactions, resulting in inappropriate industrialization. Further in (1) and (2), only long chain polynucleotide can be synthesized and (3) provides only short chain polynucleotide and these enzymes involve a defect that chain length cannot be controlled.

Further in the conventional process, yield is low; when a production amount is wished to increase, a scale of reaction tank becomes large. Furthermore, polynucleotide is purified after denaturation and inactivation of PNPase so that PNPase is disposed after use to cause high costs in using enzyme. In addition, the process encounters a shortcoming that it was impossible to completely remove contamination of synthesized poly I or poly C with PNPase protein.

(Means for solving the Problem)

The gist of the present invention lies in immobilizing PNPase to a specific carrier, that is, porous chitosan beads and further lies in producing polynucleotide using the immobilized enzyme.

In order to achieve the objects described above, the present inventors have made extensive investigations on technique for immobilizing PNPase. As a result, it has been found that by immobilizing PNPase to porous chitosan beads, PNPase can be adsorbed and immobilized extremely effectively and high activity can be maintained and exhibited. It has also been found that by using this immobilized enzyme polynucleotide can be produced in a good yield at low costs. It has further been found that continuous production of polynucleotide can be made also in a column type reactor, a hydraulic pressure type bioreactor, etc. The present invention has thus come to be accomplished.

Hereafter the present invention is described in detail.

As PNPase used in the present invention, those derived from animal or plant tissue or mycelia (including spore) of microorgansim, etc. PNPase may be purified or crude one. As PNPase derived from bacteria, for example, Micrococcus luteus, Escherichia coli, Azotobactar vinelandii, Bacillus stearothermophilus, etc. are preferred. PNPase can be obtained from its enzyme source by a known method.

The porous chitosan beads which are carriers for immobilization used in the present invention are those obtained by deacetylating chitin of Crustacea origin and, grinding and rendering the resulting chitosan porous to increase its surface area and enhance adsorbability. Taking pressure loss, particle surface area, etc. into account, a particle diameter of the porous chitosan beads is preferably 0.5 to 3.0 mm, more preferably 0.5 to 1.5 mm. Examples include CHITOPEARLE BCW-3010, BCW-3510, BCW-3505, etc.

Examples of the nucleoside-5′-diphosphate used in the present invention include cytidine-5′-diphosphate (CDP), inosine-5′-diphosphate (IDP), adenosine-5′-diphosphate (ADP), guanosine-5′-diphosphate (GDP), uridine-5′-diphosphate (UDP), thymidine-5′-diphosphate (TDP), etc. Furthermore nucleoside-5′-diphosphates having a substituent(s) may also be used.

Any crosslinking agent is usable so long as it is capable of binding to the amino group of chitosan beads upon contact with the chitosan beads. For example, glutaraldehyde, carbodiimide, etc. can be used.

There is no particular limitation to immobilization of PNPase to the porous chitosan bead carrier and there may be used a method which comprises contacting enzyme with the carrier, for example, in water or a suitable buffer solution. As the buffer solution, a phosphate buffer solution, a borate buffer solution, a Tris buffer solution, glycine-NaOH, etc. can be used. Buffer solutions having a molar concentration of 0.05 to 0.1 and showing pH of approximately 6 to 10 are particularly preferred. The amount of the carrier for enzyme is generally 0.01 to 50% (W/W), preferably 0.5 to 30% (W/W). By way of an example, PNPase is adsorbed and immobilized onto the carrier by sufficiently equilibrating 2 ml (V/V) of CHITOPEARL BCW-3510 with 10 to 100 mM buffer solution (pH 6 to 10), then adding 5 to 30 mg of a PNPase solution to 1 ml (V/V) of the carrier, and subjecting the mixture to a reciprocating shake treatment (60 to 80 strokes/min, a stroke width of 2.5 cm) for 0.5 to 24 hours at 4 to 30° C, preferably at room temperature. The unadsorbed PNPase is removed by filtering and washing on a glass filter.

The immobilized enzyme sufficient for use can be used by this method but using the carrier pretreated with a crosslinking agent, the immobilized enzyme can be obtained more advantageously. As the pretreatment, there may be used a method which comprises contacting the carrier with a crosslinking agent in water or in the buffer solution as described above. It is preferred that a concentration of the crosslinking agent be generally in the range of 0.01 to 5% based on the carrier. For example, 2 ml (v/V) of CHITOPEARLE BCW-3010 is immersed in 4 ml of 2.5% (V/V) glutaraldehyde (hereafter simply referred to as GLA) solution and the mixture is settled or shaken (30 to 80 strokes/min, a stroke width of 2.5 cm) for 0.5 to 3 hours at room temperature. After treating with GLA, an excess of GLA is removed and washed on a glass filter (G-2).

GLA-treated CHITOPEARL BCW-3010 is immobilized with enzyme in a buffer solution in a concentration of 5 to 30 mg PNPase/ml carrier, as described above. Also in the case of immobilization, settling or shaking is performed under conditions similar to those in the treatment with GLA thereby to immobilize enzyme. The enzyme which is not immobilized is washed on a glass filter or washed by shaking, until no elution of protein is noted. When immobilized as described above, an apparent rate of immobilization is calculated according to the following equation.

$$\frac{\text{Total amount of protein tested} - \text{Amount of unadsorbed protein}}{\text{Total amount of protein tested}} \times 100$$

As the result, the apparent rate for immobilization was more than 40% as will be later described.

Further as a method for immobilization of enzyme, in addition to the method described above, there may be adopted a method which comprises packing the porous chitosan bead carrier in a column, then passing 2.5% (V/V) GLA solution through the column for 2 to 3 hours, thoroughly washing with sterile distilled water and then passing a protein solution through the column.

The process for producing polynucleotide using the immobilized enzyme in accordance with the present invention is not particularly limited but polynucleotide can be produced efficiently, for example, by packing the immobilized enzyme into a column and continuously passing as a substrate 10% nucleoside through the column at pH of 8 to 9 and a temperature of 30 to 50° C.

In the case of performing by the column process, the substrate may also be passed through the column after packing the carrier in a column, running water or buffer containing the crosslinking agent down through the column and then passing a PNPase solution through the column.

The immobilized enzyme formed in the present invention can also be utilized, needless to say, in the batch system for performing the reaction in such a state that the immobilized enzyme is held on a support in a reaction tank.

The thus obtained polynucleotide can be isolated and purified in a conventional manner (for example, ultrafiltration, dialysis, etc.).

The thus obtained immobilized enzyme of the present invention has almost the same properties as those of native enzyme. However, a tendency that the optimum pH of the reaction tends to incline to an acidic area is noted.

(Examples)

Hereafter the present invention is described in more detail by referring to examples and test examples.

In these examples, the concentration of polynucleotide was determined by HPLC under the following conditions.

Column: ASAHI PACK GS-510 (6 x 500 mm)

Mobile phase: 0.1 M Tris-hydrochloride buffer (pH 7.5, 0.3 M NaCl, 1 mM EDTA)

The conditions for HPLC used for determining a molecular size were as follows.

Column: ASAHI PACK GS-710 (6 x 500 mm)

Mobile phase: 0.1 M Tris-hydrochloride buffer (pH 7.5, 0.3 M NaCl, 1 mM EDTA)

Detection: UV method (270 nm/poly C, 251 nm/poly I

Example 1

(1) Immobilization of PNPase

CHITOPEARLE BCW-3010 which had been grain-dressed and sterilized in an autoclave were taken in 2 ml (V/V) and collected through a glass filter (G-2). After thoroughly removing water, the beads were added to 4 ml of PNPase solution (0.1 M Tris-hydrochloride, pH 8.1, protein concentration of 30 mg/ml); PNPase made by Pharmacia Co. The mixture was shaken (70 strokes/min) at a low temperature (4 to 6° C) overnight (16 hours) to carry out immobilization. Washing by shake was repeated until the unbound protein was not detected.

(2) Synthesis of poly C

To 10 ml of 10% CDP solution (0.1 M Tris-hydrochloride, pH 9.0, 40 m M $MgCl_2$, 0.4 mM EDTA) was added 2 ml of the immobilized carrier followed by reacting at 37° C for 48 hours. After completion of the reaction, the amount of poly C was determined and this immobilized enzyme was washed with distilled water. The reaction was repeated and its stability was determined. The results are shown in the table.

4

| Number of Reaction | 1 | 5 | 10 | 15 | 20 |
|---|---|---|---|---|---|
| Relative Activity (%) | 100[1] | 85 | 82 | 75 | 71 |

[1] The amount (440 mg) of poly C synthesized for 48 hours was made 100.

Example 2

(1) Treatment of Carrier with GLA

CHITOPEARL BCW-3010 was used as the carrier for immobilization. After sterilizing the beads in an autoclave, 2 ml (V/V) of the carrier was taken and added to 6 ml of 2.5% (V/V) GLA aqueous solution. The mixture was shaken (70 strokes/min and a stroke width of 2.5 cm) at room temperature for 2.5 hours. After the treatment, the carrier treated with GLA was collected on a glass filter and washing by shake was repeated 5 times.

(2) Immobilization of PNPase

The carrier treated with GLA as described above was added to 4 ml of PNPase solution (0.1 M Tris-hydrochloride, pH 8.1, protein concentration of 15 mg/ml; PNPase made by Pharmacia Co.). The mixture was shaken (50 strokes/min, a stroke width of 2.5 cm) at room temperature for 15 hours to carry out immobilization. After the treatment with enzyme, the unbound enzyme was shake-washed under the same conditions as the shaking for immobilization until the unbound protein was not detected in the washing liquid. The thus obtained immobilized enzyme immobilized apparently 17 mg of PNPase per 1 ml of carrier.

(3) Synthesis of poly C

To 10 ml of 10% CDP solution (0.1 M Tris-hydrochloride, 40 mM $MgCl_2$, 0.4 mM EDTA, pH 9.0) was added 2 ml of the immobilized carrier followed by shaking (70 strokes/min, a stroke width of 2.5 cm) at 37°C. Thus poly C was synthesized. The concentration of poly C was measured with passage of time. The results are shown in the table.

| Reaction Time (hour) | 12 | 24 | 36 | 48 |
|---|---|---|---|---|
| Amount of poly C formed (mg) | 120 | 310 | 380 | 440 |

As is clear from the table, 440 mg of poly C was obtained from 1000 mg of CDP in 48 hours. A molecular size of the obtained poly C was determined by HPLC. It reveals that poly C and a molecular size of more than 12S (sedimentation constant) in the reaction time for 24 hours (molecular weight: more than 1 x $10^7$ daltons), 12S for 36 hours, 10S for 48 hours and 8S for 60 hours.

Example 3

(1) Immobilization of PNPase

CHITOPEARL BCW-3010, 2 ml (V/V), treated in a manner similar to Example 2 was added to 4 ml of PNPase solution (25 M Tris-hydrochloride, pH 8.5, protein concentration of 2 mg/ml; PNPase made by Unitika Co.). The mixture was shaken at room temperature for 15 hours to carry out immobilization. After the treatment with enzyme, the unbound enzyme was removed and washed by shaking. The obtained immobilized enzyme immobilized apparently 1.9 mg of PNPase per 1 ml of carrier.

(2) Synthesis of poly I

To 6 ml of 10% (W/V) IDP solution (0.1 M Tris-hydrochloride, 5 mM MnCl$_2$, 0.4 mM EDTA, pH 8.5) was added 2 ml of the immobilized carrier obtained in a manner similar to (1) described above. By shaking at 35°C, poly I was synthesized. Its concentration was measured with passage of time. The results are shown in the table.

| Reaction Time (hour) | 6 | 12 | 15 | 18 |
|---|---|---|---|---|
| Amount of poly I formed (mg) | 89 | 152 | 213 | 252 |

By the reaction for 18 hours, 252 mg of poly I was obtained from 600 mg of IDP. A molecular size of the obtained poly I was determined by HPLC. In the reaction time for 8 hours, poly I had a molecular size of more than 12S but showed 12S for 12 hours. Thereafter, the molecular size became smaller by 1S as the reaction time passed by 2 hours and the S value of poly I obtained by reacting for 18 hours was more than 9S.

Example 4 Synthesis of poly A

poly A was synthesized using 2 ml of the same immobilized enzyme prepared in Example 2, by mixing the enzyme with 10 ml of 10% ADP solution (0.1 M Tris-hydrochloride, pH 9.0, 40 mM MgCl$_2$, 0.4 mM EDTA) and shaking (70 strokes/min, a stroke width of 2.5 cm) at 37°C. Fourty eight hours after, poly A in the reaction solution was quantitatively determined by HPLC and 400 mg of poly A was synthesized.

Example 5 Synthesis of poly T

Poly T was synthesized by mixing 2.0 ml of the same immobilized enzyme prepared in Example 3 with 10 ml of 10% TDP solution (0.1 M Tris-hydrochloride, pH 8.5, 20 mM manganese sulfate, 0.4 mM EDTA) and shaking (70 strokes/min, a stroke width of 2.5 cm) at 35°C for 15 hours. As the result, 330 mg of poly T was obtained.

Example 6 Synthesis of poly C by bioreactor

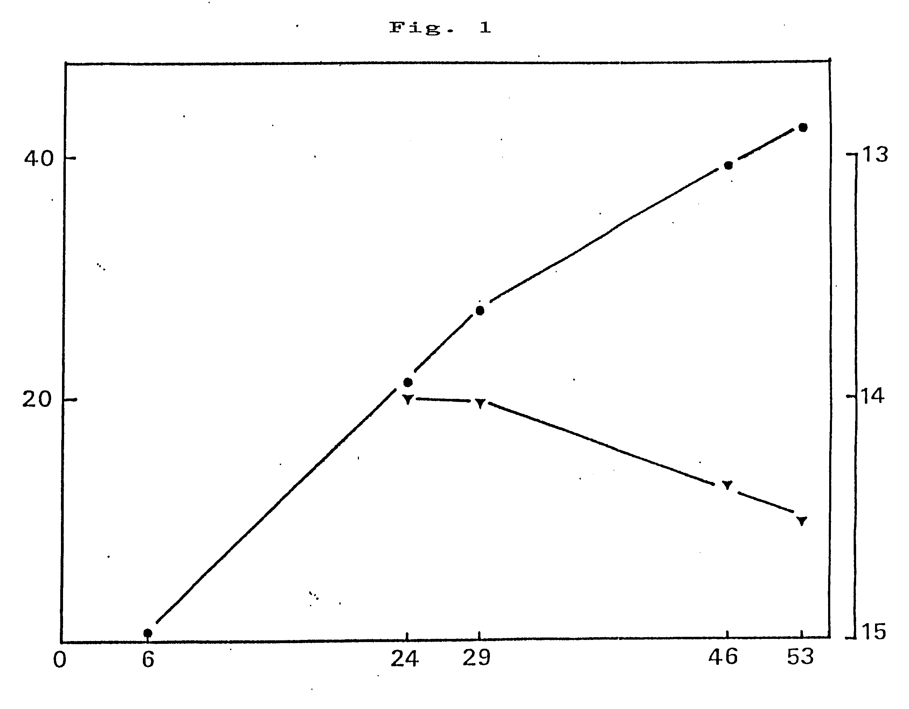

By the same manner as in Example 2, 100 ml (V/V) of the immobilized enzyme was prepared. The immobilized enzyme is charged in SAKURA Bioreactor TBR-2 (manufactured by SAKURA SEIKI) and 600 ml of 10% CDP (0.1 M Tris-hydrochloride, pH 9.0) solution was added thereto. In a large scale poly C was synthesized at an agitation of 200 rpm, a temperature of 37 ± 0.5°C. The results are shown in Fig. 1. The amount of poly C produced reached 24.7 g in 53 hours. This reveals that the activity is sufficiently exhibited also in the batch system hydraulic bioreactor, showing that synthesis can be continuously made in a large scale.

Example 7 Preparation of PNPase

In 250 ml of 0.5% aqueous saline solution was suspended 25 g of Micrococcus luteus (Micrococcus lyzodicticus ) ATCC 4698 dry cells (manufactured by Sigma Co.). The suspension was rendered pH of 8.0 with 1N sodium hydroxide and 100 mg of lysozyme was added to the suspension followed by incubation at 37°C for 30 minutes. After ice-cooling, centrifugation was performed at 20,000 x g for 30 minutes and the supernatant was taken. Ammonium sulfate was added to the supernatant to 33% saturation. After allowing to stand for 30 minutes, centrifugation was performed and the supernatant was obtained. An equal volume of saturated ammonium sulfate (pH 8.0) to the supernatant was added and the mixture was thoroughly mixed.

After allowing to stand for 30 minutes, centrifugation was performed. The precipitates were dissolved in 37 ml of 0.1 M Tris-HCl buffer (pH 8.1, 1 mM 2-mercaptoethanol) and the solution was dialyzed to the buffer. After the dialysis, the dialysate was diluted with 0.1 M Tris buffer (pH 8.1) in a protein concentration of 10 mg/ml. Then, 24.4 g of ammonium sulfate powders were added per 100 ml followed by centrifugation at 20,000 x g for 30 minutes. The supernatant was taken and 8.7 g of ammonium sulfate powders were added per 100 ml followed by centrifugation. The precipitates were dissolved in 40 ml of 0.1 M Tris-HCl buffer (pH 8.1, 10 mM 2-mercaptoethanol, 1 mM EDTA) and the solution was dialyzed to the buffer to give 750 mg of crude PNPase fraction. This crude PNPase was treated in a manner similar to Example 2 to prepare immobilized enzyme. Using 2 ml of this immobilized enzyme, poly C was synthesized in a manner similar to Example 2. The results are shown in the following table.

| Time (hour) | 12 | 24 | 36 | 48 |
|---|---|---|---|---|
| Amount of poly C (mg) | 180 | 360 | 421 | 532 |

It is also possible to immobilize the crude PNPase extracted from the cells to CHITOPEARL BCW-3010 (treated with GLA). It was noted that its polymer synthesizing capability was extremely high.

Example 8.

Two ml of immobilized enzyme was prepared by the same manner as in Example 2. This immobilized enzyme (2 ml) was placed in a 250 ml Erlenmeyer flask, then 50 ml of 7.5% CDP solution (0.2M Tris-HCl buffer, pH 9.50, 25 mM $MgCl_2$, 0.4mM EDTA) was added, and the mixture was agitated at 30°C to synthesize poly C. The result is given in the following table.

| Reaction Time (hr) | 12 | 24 | 48 | 72 | 96 | 120 |
|---|---|---|---|---|---|---|
| Poly C Chain Length (min) | 13.3 | 14.5 | 15.2 | 16.2 | 17.0 | 17.8 |

It is apparent from the table that the chain length or molecular weight of the synthesized poly C becomes smaller as the reaction time goes on though such a change is as slow as 0.5-1.0 min/24 hr. This shows that poly C of various molecular weights can be synthesized using this immobilized enzyme by changing the reaction time. Further, the substrate for the reaction solution was changed to IDP so that poly I of various sizes were synthesized as well.

Test Example 1 Enzyme Activity in Synthesis of Poly C

To 990 μl of 10% (W/V) CDP solution (0.1 M Tris-hydrochloride, 40 mM $MgCl_2$, 0.4 mM EDTA, pH 9.0) was added 10 μl of enzyme solution (1.6 mg PNPase, manufactured by PL-Pharmacia Co., Product No. 27-0302) followed by reacting at 37°C for 16 hours. After completion of the reaction, an amount of poly C formed was determined and an amount of enzyme for synthesizing 1 mg of poly C in an hour was made 1 unit. Native enzyme (Pharmacia Co., No. 27-0302) showed a specific activity of 2.34 units/mg protein. The enzyme activity in the present invention was determined by the ability of synthesizing polynucleotide by reacting for 16 hours.

Test Example 2 Enzyme Activity in Synthesis of Poly I

To 990 μl of 10% (W/V) IDP solution (0.1 M Tris-hydrochloride, 5 mM $MnCl_2$, 0.4 mM EDTA, pH 8.5) was added 10 μl of enzyme solution (0.1 mg protein, manufactured by Unitika Co.) followed by reacting at 37°C for 17 hours. After completion of the reaction, an amount of poly I formed was determined and an amount of enzyme for synthesizing 1 mg of poly I in an hour was made 1 unit. Native enzyme showed a specific activity of 32.9 units/mg protein.

Test Example 3

Immobilized enzyme was prepared under reaction conditions similar to Example 2 in a manner similar to Example 2. The reaction for synthesizing poly C was continuously repeated to examine stability of the immobilized enzyme. The results are shown in the table.

| Number of Reaction | 1 | 10 | 15 | 20 | 30 |
|---|---|---|---|---|---|
| Amount of poly C formed (mg) | 435 | 420 | 425 | 415 | 395 |

Even though the continuous batch reaction was repeated 30 times, the activity was retained in more than 90% of the initial activity, showing that the immobilized enzyme had a high stability.

Test Example 4

Immobilized enzyme was prepared in a manner similar to Example 3. The reaction for synthesizing poly I was continuously repeated under the same reaction conditions as in Example 3 to examine stability of the immobilized enzyme. The results are shown in the table.

| Number of Reaction | 1 | 10 | 20 | 30 | 40 |
|---|---|---|---|---|---|
| Amount of poly I formed (mg) | 252 | 230 | 216 | 220 | 210 |

As is clear from the table, the activity was retained in 83% of the initial ctivity, showing that the immobilized enzyme had a high stability.

Test Example 5

An amount of endotoxin in polynucleotide synthesized was determined using a toxycolor test (Biochemical Industry). The amount of endotoxin in poly C obtained in Example 2 was 0.02 to 0.5 E.U./mg and the amount of endotoxin in poly I obtained in Example 3 was 0.004 to 0.05 E.U./mg.

(Effects of the Invention)

As is evident from the foregoing facts, by effectively immobilizing PNPase to the porous chitosan beads, (1) a stable immobilized enzyme can be obtained substantially without inactivating the enzyme activity, (2) the activity of PNPase can be maintained over long periods of time and PNPase is stable, (3) the immobilized enzyme can be continuously used without disposing after use, (4) using the immobilized enzyme, a production efficiency of polynucleotide can be enhanced, (5) foreign protein contaminant can be prevented from being mixed with the synthesized polynucleotide and further pyrogen can be removed to save energy in the step of purification so that polynucleotide of high quality can be synthesized in a good yield. Furthermore, (6) it is unnecessary to denature or inactivate PNPase so that polynucleotide can be continuously synthesized in a large scale in a column type reactor or a hydraulic pressure type bioreactor, without a reaction tank of large volume, etc. Furthermore, (7) it was quite unexpected but by changing the reaction time, polynucleotide having an optional molecular size can be obtained.

4. Brief Description of the Drawing

Fig. 1 shows synthesis of poly C using bioreactor TBR-2. The ordinate shows poly C concentration (mg/ml) and retention time (min) and the abscissa shows reaction time, wherein ● denotes a concentration of poly C and ▼ denotes a molecular size.

## Claims

(1) An immobilized enzyme comprising porous chitosan beads having immobilized thereto polynucleotide phosphorylase.

(2) A process for producing polynucleotide characterized by using an immobilized enzyme as claimed in claim 1.

(3) An immobilized enzyme comprising porous chitosan beads, which undergo a crosslinking treatment with a crosslinking agent, having immobilized thereto polynucleotide phosphorylase.

(4) A process for producing polynucleotide characterized by using an immobilized enzyme as claimed in claim 3.

Fig. 1

EP 0 368 808 A2